# EUROPÄISCHE PATENTSCHRIFT

(11) **EP 2 188 250 B1**
(45) Veröffentlichungstag und Bekanntmachung des Hinweises auf die Patenterteilung: **19.10.2011**
(21) Anmeldenummer: 08803382.4
(22) Anmeldetag: 29.08.2008
(51) Int. Cl.: C07C 403/24, A23L 1/275, A23K 1/16

(54) **VERFAHREN ZUR HERSTELLUNG VON ÖLIGEN LÖSUNGEN VON ASTAXANTHIN-DERIVATEN**
METHOD FOR PRODUCING OIL-CONTAINING SOLUTIONS OF ASTAXANTHIN DERIVATIVES
PROCÉDÉ DE PRODUCTION DE SOLUTIONS HUILEUSES DE DÉRIVÉS DE L'ASTAXANTHINE

(30) Priorität: 31.08.2007 EP 07115469
(43) Veröffentlichungstag der Anmeldung: 26.05.2010
(73) Patentinhaber: BASF SE, 67056 Ludwigshafen (DE)
(72) Erfinder: FELDTHUSEN JENSEN, Jesper, 55268 Nieder-Olm (DE); KÖPSEL, Christian, 69469 Weinheim (DE); PFEIFFER, Angelika-Maria, 67466 Lambrecht (DE); ERNST, Hansgeorg, 67346 Speyer (DE)
(86) Internationale Anmeldenummer: PCT/EP2008/061384
(87) Internationale Veröffentlichungsnummer: WO 2009/027499

(56) Entgegenhaltungen:
- EP-A- 1 952 845
- WO-A-03/066583
- WO-A-2006/125591

## Beschreibung

Die vorliegende Erfindung betrifft die Herstellung von Lösungen von Astaxanthin-Derivaten in essbaren Ölen sowie die durch dieses Verfahren erhältlichen Lösungen des Astaxanthin-Derivats in einem essbaren Öl.

Astaxanthin (3,3'-Dihydroxy-β,β-carotin-4,4'-dion) und seine Derivate, insbesondere seine Ester sind als Nahrungs- und Futtermittelzusatzstoffe von Interesse. So wird Astaxanthin in großem Umfang bei der Aufzucht von Speisefischen in Aquakulturen (Fischfarmen) als Futtermittelzusatz eingesetzt. Aufgrund der vitaminartigen Eigenschaft von Astaxanthin und seinen Derivaten wirken sie sich vorteilhaft auf die Gesundheit der Fische in den Aquakulturen aus und fördern deren Fruchtbarkeit. Zudem bewirken Astaxanthin uns seine Derivate eine Intensivierung der Färbung von Fleisch und Haut der Fische, was nicht zuletzt aus ästhetischen und kulinarischen Gründen wünschenswert ist.

Problematisch sind die geringe Wasserlöslichkeit von Astaxanthin und seinen Derivaten und ihre damit einhergehende schlechte Bioverfügbarkeit. Aus diesem Grund können Astaxanthin und seine Derivate nicht als solche eingesetzt werden, sondern müssen in eine Formulierung überführt werden, die eine hinreichende Bioverfügbarkeit dieser Substanzen gewährleistet. Aufgrund der chemischen Instabilität vom Astaxanthin und seinen Derivaten stellt jedoch die Formulierung dieser Verbindungen eine besondere Herausforderung dar. Hierbei sind flüssige Formulierungen des Astaxanthins in essbaren, d. h. für Nahrungsmittel zugelassenen Ölen von besonderem Interesse.

Die EP 1213013 A beschreibt ein Verfahren zur Herstellung öliger Suspensionen des Astaxanthins, bei dem man zunächst ein Trockenpulver des Astaxanthins, welches ein oder mehrere Schutzkolloide enthält, durch Mahlen auf eine mittlere Partikelgröße von maximal 100 µm zerkleinert und anschließend die Partikel in einem geeigneten Öl suspendiert. Die Herstellung des Astaxanthin enthaltenden Trockenpulvers erfolgt durch Lösen oder Dispergieren des Astaxanthins in einer wässrigen Lösung des Schutzkolloids, Ausflocken des Schutzkolloids zusammen mit dem Astaxanthin und Abtrennen des ausgeflockten Feststoffs. Das Verfahren ist vergleichsweise aufwendig.

Die WO 96/23420 beschreibt die Herstellung von öligen Astaxanthin-Suspensionen, worin die Partikel des Astaxanthins im Wesentlichen Teilchengrößen < 2 µm aufweisen, durch Vermahlen von Astaxanthin in einem Öl. Die Astaxanthinpartikel in dieser Suspension neigen zur Agglomeration.

Die WO 03/102116 beschreibt ölige Lösungen von Carotinoiden wie Astaxanthin. Ihre Herstellung erfolgt durch Lösen des Carotinoids in einem organischen Lösungsmittel wie N-Methylpyrrolidon in Anwesenheit eines lipophilen Dispergiermittels und Entfernen des Lösungsmittels. Das erhaltene Pulver wird dann in einem Öl, beispielsweise Fischöl, gelöst. Auch dieses Verfahren ist vergleichsweise aufwendig und bedarf größerer Mengen an Schutzkolloiden.

Die WO2006/125591 beschreibt die Herstellung von öligen Carotinoid-Lösungen, beispielsweise Lösungen des Astaxanthins, bei dem man zunächst eine Suspension des Carotinoids in der Ölphase bereitstellt, die Suspension für eine kurze Zeit auf hohe Temperaturen, z. B. im Bereich von 100 bis 230°C erhitzt, um das Carotinoid in Lösung zu bringen, und die heiße Lösung mit einem kalten Öl vermischt. Dieses Verfahren ist mit einer Reihe von Nachteilen verbunden. Zum Einen führt das Erhitzen aufgrund der chemischen Labilität von ß-Carotinoiden unter anderem zu einer unerwünschten cis/trans-Isomerisierung der exocyclischen Doppelbindungen und damit zu einem Aktivitätsverlust. Zudem erweist sich die Bereitstellung öliger Suspensionen des Astaxanthins durch Vermahlen von Astaxanthin in Öl als problematisch.

Der vorliegenden Erfindung liegt die Aufgabe zu Grunde, ein Verfahren zur Herstellung einer Zusammensetzung von Astaxanthin oder einem Astaxanthin-Derivat in einem essbaren zugelassenen Öl bereitzustellen, welches die Nachteile des Standes der Technik vermeidet.

Es wurde überraschenderweise gefunden, dass die im folgenden beschriebenen Astaxanthin-Derivate der allgemeinen Formel I sowie Astaxanthin-Derivate, die zu wenigstens 70 Gew.-% aus den Verbindungen der Formel I bestehen, nicht nur eine gute Löslichkeit in essbaren Ölen aufweisen, sondern zudem auch eine vorteilhafte Lösungskinetik besitzen, d. h. sich rasch in einem solchen Öl auflösen, wenn man sie in Form einer Suspension in einem solchen essbaren Öl bereitstellt, worin die Partikel des Astaxanthin-Derivats einen volumenmittleren Teilchendurchmesser im Bereich von 0,5 bis 50 µm aufweisen (bestimmt durch Fraunhofer-Beugung).

Hierbei handelt es sich um Astaxanthin-Derivate der folgenden Formel I 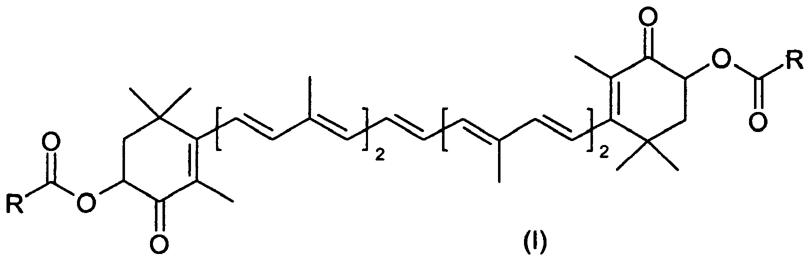 worin R für einen Rest der Formel 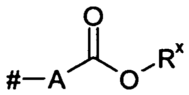 steht, worin # die Anknüpfung an die Carbonylgruppe bedeutet, A für lineares oder verzweigtes C₁-C₆-Alkylen oder für -CH=CH- steht und R^{x} für C₁-C₁₀ Alkyl steht.

Derartige Astaxanthin-Derivate und Verfahren zu ihrer Herstellung sind aus der WO 03/066583 A1 bekannt.

Demnach betrifft die vorliegende Erfindung ein Verfahren zur Herstellung einer Lösung eines Astaxanthin-Derivats in einem essbaren Öl, umfassend
i) Bereitstellung einer Suspension eines Astaxanthin-Derivats in einem essbaren Öl, wobei die Partikel des Astaxanthin-Derivats in der Suspension einen volumenmittleren Teilchendurchmesser, bestimmt durch Fraunhofer-Beugung, im Bereich von 0,5 bis 50 µm aufweisen, und
ii) Einarbeiten der Suspension des Astaxanthin-Derivats in ein essbares Öl, wobei man eine Lösung des Astaxanthin-Derivats der Formel I erhält,
wobei das Astaxanthin-Derivat zu wenigstens 70 Gew.-% aus wenigstens einer Verbindungen der Formel I, wie hier beschrieben, besteht.

Das erfindungsgemäße Verfahren ist mit einer Reihe von Vorteilen verbunden. Zum einen lassen sich auf diese Weise Lösungen von Astaxanthin-Derivaten in essbaren Ölen mit hoher Konzentration von bis zu 2 Gew.-%, bezogen auf das Gesamtgewicht der Lösung, herstellen. Zudem zeichnen sich die Lösungen durch einen sehr hohen Anteil an all-trans-Isomeren aus, der in der Regel oberhalb 80 %, insbesondere oberhalb 90 %, bezogen auf das in der Formulierung enthaltene Astaxanthin-Derivat liegt. Ferner lassen sich ölige Suspensionen von Astaxanthin-Derivaten, die zu wenigstens 70 Gew.-% aus wenigstens einer Verbindungen der Formel I bestehen, sehr viel einfacher durch Vermahlen herstellen als ölige Suspension des Astaxanthins selber oder anderer, davon verschiedener Astaxanthin-Derivate wie Astaxanthin-Diacetat, Astaxanthindisuccinat oder Astaxanthin-Dipalmitat.

In Formel I steht C₁-C₁₀-Alkyl für einen linearen oder verzweigten, gesättigten Kohlenwasserstoffrest mit 1 bis 10, insbesondere 1 bis 6 und speziell 1 bis 4 C-Atomen wie Methyl, Ethyl, n-Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, und 1-Ethyl-2-methylpropyl.

C₁-C₆-Alkylen steht für einen linearen oder verzweigten, bivalenten, gesättigten Kohlenwasserstoff-Rest mit 1 bis 6 C-Atomen und insbesondere 2 bis 4 C-Atomen wie Methylen, 1,2-Ethandiyl, 1,1-Ethandiyl, 1,3-Propandiyl, 1,2-Propandiyl, 1-Methylpropan-1,2-diyl, 2-Methylpropan-1,2-diyl, 2-Methylpropan-1,3-diyl, 2,2-Dimethylpropan-1,3-diyl, 1,4-Butandiyl, 2-Methylbutan-1,4-diyl, 1,5-Pentandiyl oder 1,6-Hexandiyl.

Die beiden Reste R in Formel I können gleich oder verschieden sein und sind vorzugsweise identisch.

Vorzugsweise steht A für C₂-C₄-Alkylen und insbesondere für 1 ,2-Ethandiyl. R^{x} steht vorzugsweise für C₁-C₆-Alkyl, insbesondere C₁-C₄-Alkyl und speziell für Methyl oder Ethyl.

Verbindungen der Formel I, worin A im Rest R für 1,2-Ethandiyl steht, werden im Folgenden auch als Dialkyldisuccinate bezeichnet. Hierunter besonders bevorzugt ist das Dimethylsuccinat.

Unter einem essbaren Öl versteht man im Sinne der Erfindung ein für Nahrungsmittel, d. h. ein für die Tier- und/oder Humanernährung zugelassenes Öl. Der Begriff "Nahrungsmittel" umfasst hier und im Folgenden Nahrungsmittel für die Humanemährung (= Lebensmittel) und Nahrungsmittel für die Tierernährung (= Futtermittel). Das essbare Öl kann synthetischer, mineralischer, pflanzlicher oder tierischer Herkunft sein. Beispiel sind Pflanzenöle wie Sojabohnen-Öl, Palmöl, Palmkernöl, Sonnenblumenöl, Maiskeimöl, Leinsamenöl, Rapsöl, Distelöl, Weizenkeimöl, Reisöl, Kokosöl, Mandelöl, Aprikosenkernöl, Avocadoöl, Jojobaöl, Haselnussöl, Walnussöl, Erdnussöl, Pistazienöl, Triglyceride mittelkettiger (= C₈-C₁₀) pflanzlicher Fettsäuren (sog. MCT-Öle) und PUFA-Öle (PUFA = mehrfach ungesättigte Fettsäuren (polyunsaturated fatty acids) wie Eikosapentaensäure (EPA), Docosahexaensäure (DHA) und α-Linolensäure), weiterhin semisynthetische Triglyceride, z.B. Caprylsäure/Caprinsäure-Tricyceride wie die Miglyol-Typen, weiterhin Oleostearin, Paraffinöl, Glycerylstearat, Isopropylmyristat, Diisopropyladipat, 2-Ethylhexansäureacetylstearylester, flüssige hydrierte Polyisobutene, Squalan, Squalen, weiterhin tierische Öle und Fette wie Fischöle einschließlich Makrelen-, Sprotten-, Thunfisch-, Heilbutt-, Kabeljau- und Lachsöl und Lanolin.

Bevorzugt sind essbare Öle, die bei 25 °C flüssig sind, insbesondere Pflanzenöle wie Sojabohnen-Öl, Palmöl, Palmkernöl, Sonnenblumenöl, Distelöl, Maiskeimöl, Olivenöl, Leinsamenöl, Rapsöl, Reisöl, Kokosöl, Erdnussöl, PUFA-Öle, MCT-Öle, weiterhin Fischöle, sowie Mischungen dieser Öle.

In einer besonders bevorzugten Ausführungsform der Erfindung erfasst das im erfindungsgemäßen Verfahren eingesetzte, essbare Öl zu wenigstens 50 Gew.-%, insbesondere zu wenigstens 80 Gew.-%, bezogen auf die Gesamtmenge der in den Schritten i) und ii) eingesetzten essbaren Öle wenigstens ein unter Palmkernöl und MCT-Ölen ausgewähltes essbares Öl.

In einer anderen bevorzugten Ausführungsform der Erfindung umfasst das im erfindungsgemäßen Verfahren eingesetzte essbare Öl zu wenigstens 50 Gew.-% und insbesondere zu wenigstens 80 Gew.-%, bezogen auf die Gesamtmenge des in den Schritten i) und ii) eingesetzten essbaren Öls, wenigstens ein unter Maisöl, Sonnenblumenöl, Sojabohnenöl und Fischöl ausgewähltes essbares Öl.

Bei den essbaren Ölen kann es sich um Raffinatöle oder Rohöle handeln, die noch ursprungsspezifische Verunreinigungen wie Proteine, Phosphat, (Erd)alkalimetallsalze und dergleichen in üblichen Mengen enthalten. Die im erfindungsgemäßen Verfahren eingesetzten Öle können auch geringe Mengen an emulgiertem Wasser, vorzugsweise jedoch nicht mehr als 10 Gew.-%, insbesondere nicht mehr als 1 Gew.%, und speziell nicht mehr als 0,1 Gew.-%, bezogen auf die Gesamtmenge des eingesetzten essbaren Öls, enthalten.

In Schritt i) des erfindungsgemäßen Verfahrens wird eine Suspension des Astaxanthin-Derivats in dem essbaren Öl bereitgestellt, wobei die Partikel des Astaxanthin-Derivats in der Suspension einen volumenmittleren Teilchendurchmesser im Bereich von 0,5 bis 50 µm, insbesondere im Bereich von 0,7 bis 30 µm und speziell im Bereich von 1 bis 20 µm aufweisen. Ganz speziell liegt der volumenmittlere Teilchendurchmesser im Bereich von 1,2 bis 10 µm.

Unter dem volumenmittleren Teilchendurchmesser versteht man den mittels Fraunhofer-Beugung an einer verdünnten 0,01 bis 0,1 Gew.-%igen, speziell 0,05 Gew.-%igen Suspension der Kristalle in dem essbaren Öl bestimmten volumenmittleren Teilchendurchmesser (D_{4,3}-Wert).

Die Bereitstellung der Suspension des Astaxanthin-Derivats der Formel I in dem essbaren Öl kann in Analogie zu bekannten Verfahren zur Herstellung öliger Suspensionen von ß-Carotinoiden erfolgen, wie sie beispielsweise in dem eingangs zitierten Stand der Technik beschrieben werden. Hierzu kommen grundsätzlich Verfahren in Betracht, bei denen ein durch Fällung oder in sonstiger Weise hergestelltes Pulver des Astaxanthin-Derivats in Öl suspendiert wird, sowie Verfahren, bei denen ein festes Astaxanthin-Derivat in dem essbaren Öl durch Vermahlen auf die gewünschte Teilchengröße gebracht wird.

Das zur Herstellung der Suspension in Schritt i) eingesetzte Astaxanthin-Derivat weist üblicherweise eine Reinheit, bezogen auf das in Formel I gezeigten all-trans-Isomer, von wenigstens 70 Gew.-%, häufig wenigstens 80 Gew.-%, vorzugsweise wenigstens 90 Gew.-% und insbesondere wenigstens 95 Gew.-% auf. Neben dem all-trans-Isomeren kann das Astaxanthin-Derivat auch Anteile an Verbindungen der Formel I enthalten, in denen eine oder mehrere Doppelbindungen der in Formel I dargestellten all-trans-Isomere cis-Konfiguration aufweisen. Die Gesamtmenge an all-trans-Isomer der Formel I und cis-Isomeren der Formel I beträgt in der Regel wenigstens 80 Gew.-%, häufig wenigstens 90 Gew.-%, insbesondere wenigstens 95 Gew.-% und speziell wenigstens 99 Gew.-%. Neben dem all-trans-Isomeren der Formel I und etwaigem Cis-Isomeren kann das zur Herstellung eingesetzte Astaxanthin-Derivat auch weitere Carotinoide enthalten, wobei der Anteil dieser Verunreinigungen in der Regel 30 Gew.-%, häufig 20 Gew.-%, insbesondere 10 Gew.-%, besonders bevorzugt 5 Gew.-% und speziell 1 Gew.-%, bezogen auf die Gesamtmenge des eingesetzten Astaxanthin-Derivats nicht überschreitet. Bei diesen Verunreinigungen handelt es sich in erster Linie um Halbester des Astaxanthins, d. h. um Verbindungen der im Folgenden gezeigten Formel II 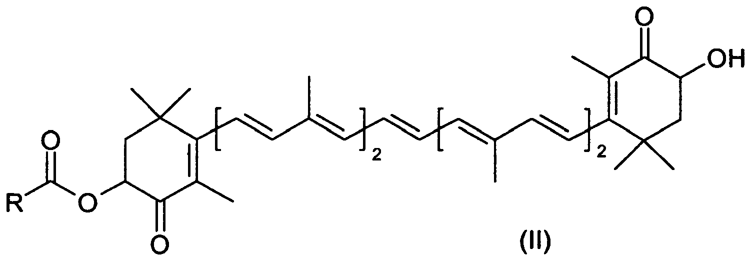 worin R die zuvor genannten Bedeutungen hat bzw. um cis-Isomere davon, sowie um Astaxanthin selber.

Insbesondere verwendet man ein Astaxanthin-Derivat, das die an ein für Nahrungsmittel (d.h. für Lebens- und/oder Futtermittel) zugelassenes Astaxanthin gestellten Anforderungen erfüllt, d. h. das weniger als 5 Gew.-% Carotinoide, die von Astaxanthin und seinen Derivaten verschieden sind, enthält und das einen Schwermetallgehalt von höchstens 10 ppm aufweist und das zu wenigstens 70 Gew.-%, häufig wenigstens 80 Gew.-%, vorzugsweise wenigstens 90 Gew.-% und insbesondere wenigstens 95 Gew.-% aus dem all-trans-Isomeren der Formel I besteht.

Das zur Herstellung der Suspension eingesetzte essbare Öl ist üblicherweise unter den vorgenannten essbaren Ölen, insbesondere unter den als bevorzugt oder besonders bevorzugt genannten essbaren Ölen ausgewählt. Das eingesetzte Öl kann geringe Mengen an Antioxidantien, z. B. Tocopherole wie α-Tocopherol oder Ascorbylpalmitat enthalten. Daneben kann das essbare Öl auch geringe Mengen an emulgiertem Wasser, vorzugsweise jedoch nicht mehr als 10 Gew.%, insbesondere nicht mehr als 1 Gew.-%, bezogen auf die Gesamtmenge des eingesetzten essbaren Öls enthalten.

Gemäß einer bevorzugten Ausführungsform erfolgt die Bereitstellung der Suspension in Schritt i) des erfindungsgemäßen Verfahrens durch Vermahlen des Astaxanthin-Derivats, welches die oben genannte Reinheit aufweist, in einem essbaren Öl. Hierzu wird man in der Regel das Astaxanthin-Derivat zunächst in dem Öl suspendieren, wobei man eine grobteilige Suspension des Astaxanthin-Derivats erhält, und anschließend die Partikel in einer geeigneten Vorrichtung zum Vermahlen auf die gewünschte Teilchengröße zerkleinern. Als Vorrichtungen zum Vermahlen können übliche, dem Fachmann bekannte Vorrichtungen, beispielsweise Kugelmühlen, Perlmühlen oder Kolloid-Mühlen eingesetzt werden.

Das Vermahlen erfolgt typischerweise bei Temperaturen unterhalb 100 °C, häufig im Bereich von 20 bis 90 °C und insbesondere im Bereich von 30 bis 70 °C. Das Vermahlen wird solange durchgeführt, bis die gewünschte Teilchengröße erreicht ist.

Die Konzentration an Astaxanthin-Derivat in der öligen Suspension wird in der Regel so gewählt, dass sie im Bereich von 1 bis 20 Gew.-%, insbesondere im Bereich von 1,5 bis 10 Gew.-% liegt. Dementsprechend wird man zum Vermahlen entsprechende Mengenverhältnisse an Öl und Astaxanthin-Derivat einsetzen.

Gegebenenfalls kann die Bereitstellung der Suspension in Gegenwart üblicher Mittel zur Stabilisierung derartiger Suspensionen wie lipophile Dispergiermittel, Antioxidantien (Oxidationsstabilisatoren) und dergleichen erfolgen. Beispiele für Antioxidantien sind Tocopherole wie α-Tocopherol, α-Tocopherolpalmitat, α-Tocopherolacetat, tert.Butylhydroxytoluol, tert.-Butylhydroxytoluol, tert.-Butylhydroxyanisol, Ascorbinsäure, deren Salze und Ester wie z.B. Natriumascorbat, Calciumascorbat, Ascorbylphosphatester und Ascorbylpalmitat und Ethoxyquin. Die Antioxidantien werden, sofern erwünscht, typischerweise in Mengen von 0,01 bis 10 Gewichtsteilen, insbesondere 0,01 bis 0,3 Gewichtsteilen, bezogen auf ein Gewichtsteil des Astaxanthin-Derivat eingesetzt. Typische lipophile Dispergiermittel sind Ascorbylpalmitat, Polyglycerin-Fettsäureester wie Polyglycerin-3-polyrizinoleat (PGPR90), Sorbitanfettsäureester wie Sorbitanmonostearat (SPAN60), PEG(20)-Sorbitolmonooleat, Propylenglycol-Fettsäureester sowie Phospholipide wie Lecithin. Lipophile Dispergiermittel werden, sofern erwünscht, üblicherweise in Mengen von 0,1 bis 10 Gew.-Teilen, bezogen auf 1 Gew.-Teil des Astaxanthin-Derivats eingesetzt. In einer bevorzugten Ausführungsform der Erfindung wird kein lipophiles Dispergiermittel eingesetzt.

Die in Schritt i) erhaltenen Suspensionen, die kein lipophiles Dispergiermittel enthalten, sind neu und ebenfalls Gegenstand der vorliegenden Erfindung. Dementsprechend betrifft die vorliegende Erfindung insbesondere eine ölige Suspension eines Astaxanthin-Derivats, bestehend aus:
a) 1 bis 20 Gew.-%, insbesondere 1,5 bis 10 Gew.-% wenigstens eines feinteiligen Astaxanthin-Derivats, das zu wenigstens 70 Gew.-%, häufig zu wenigstens 80 Gew.-%, vorzugsweise zu wenigstens 90 Gew.-%, insbesondere zu wenigstens 95 Gew.-%, speziell zu wenigstens 99 Gew.%, bezogen auf die Gesamtmenge an Astaxanthin-Derivat in der Zubereitung, aus einem Astaxanthin-Derivat der Formel I besteht,
b) 80 bis 99 Gew.-%, insbesondere 90 bis 98,5 Gew.-%, wenigstens eines essbaren Öls oder Mischung essbarer Öle, und
c) gegebenenfalls ein oder mehrere Antioxidantien,
worin die Teile des Astaxanthin-Derivats der Formel I einen volumenmittleren Teilchendurchmesser, bestimmt durch Fraunhofer-Beugung, im Bereich von 0,5 bis 50 µm, insbesondere im Bereich von 0,7 bis 30 µm, speziell im Bereich von 1 bis 20 µm aufweisen. Ganz speziell liegt der volumenmittlere Teilchendurchmesser im Bereich von 1,2 bis 10 µm. Hierbei sind alle Angaben in Gew.% auf das Gesamtgewicht der öligen Suspension bezogen.

Bezüglich Art und Menge der Antioxidantien gilt grundsätzlich das zuvor gesagt. Beispiele für Antioxidantien, die in den erfindungsgemäßen Suspensionen enthalten sein können, sind Tocopherole wie α-Tocopherol, α-Tocopherolpalmitat, α-Tocopherolacetat, tert.-Butylhydroxytoluol, tert.-Butylhydroxytoluol, tert.-Butylhydroxyanisol, Ascorbinsäure, deren Salze und Ester wie z. B. Natriumascorbat, Calciumascorbat, Ascorbylphosphatester und Ascorbylpalmitat und Ethoxyquin. Die Antioxidantien sind in den erfindungsgemäßen Suspensionen, sofern erwünscht, typischerweise in Mengen von 0,01 bis 10 Gewichtsteilen, insbesondere 0,01 bis 0,3 Gewichtsteilen, bezogen auf ein Gewichtsteil des Astaxanthin-Derivat, enthalten.

Die in Schritt i) erhaltene Suspension des Astaxanthin-Derivats wird anschließend in ein essbares Öl eingearbeitet, wobei man eine Lösung des Astaxanthin-Derivats erhält. Das Einarbeiten wird dabei so durchgeführt, dass vorzugsweise wenigstens 90 %, insbesondere wenigstens 95 % und speziell wenigstens 99 % des in Schritt i) eingesetzten Astaxanthin-Derivats molekular gelöst vorliegen.

Der Ausdruck "molekular gelöst" bezeichnet eine Lösung, in der das Astaxanthin-Derivat in molekular-disperser Form oder in Form molekularer Aggregate des Astaxanthin-Derivats (z. B. Dimere, Trimere, etc.) vorliegt. Der Grad der Lösung des Astaxanthin-Derivats in der öligen Phase kann durch Filtration der öligen Phase durch einen feinen Filter mit einer Porengröße von 0,45 µm oder 0,20 µm, gefolgt von einer HPLC-Bestimmung des Gehalts an Astaxanthin-Derivat im Filtrat und Vergleich mit dem Gesamtgehalt an Astaxanthin in der unfiltrierten öligen Phase bestimmt werden.

Das Einarbeiten der Suspension des Astaxanthin-Derivats in das essbare Öl kann in üblicher Weise durch Vermischen der Suspension mit dem Öl erfolgen, wobei man das Vermischen vorzugsweise bei eine erhöhten Temperatur, vorzugsweise oberhalb 20 °C, insbesondere bei wenigstens 30 °C durchführt.

In der Regel wird man beim Einarbeiten der Suspension in das essbare Öl eine Temperatur unterhalb 100 °C einhalten. Das Einarbeiten der Suspension in das essbare Öl erfolgt typischerweise bei Temperaturen unterhalb 100 °C, vorzugsweise bei Temperaturen von nicht mehr als 90 °C und speziell nicht mehr als 80 °C und vorzugsweise bei Temperaturen im Bereich von 30 bis 90 °C, insbesondere im Bereich von 30 bis 80 °C und speziell im Bereich von 30 bis 70 °C.

Vorzugsweise wird man das Öl, in welches man die Suspension des Astaxanthin-Derivats einarbeitet erwärmen. In der Regel liegt die Temperatur des erwärmten Öls vorzugsweise unterhalb 100 °C und beträgt insbesondere nicht mehr als 90 °C und speziell nicht mehr als 80 °C, z. B. 30 bis 90 °C, insbesondere 30 bis 80 °C und speziell 30 bis 70 °C.

Typischerweise wird man die relativen Mengen an Öl und Suspension so wählen, dass der Gehalt an Astaxanthin-Derivat in der resultierenden Lösung im Bereich von 100 ppm (= 0,01 Gew.-%) bis etwa 2 Gew.-%, häufig im Bereich von 100 ppm bis 1,8 Gew.-%, insbesondere im Bereich von 150 ppm bis 1,5 Gew.-%, bezogen auf die Gesamtmenge an Öl plus Astaxanthin-Derivat liegt. Gemäß einer ersten bevorzugten Ausführungsform wird man die relativen Mengen an Suspension und essbarem Öl so wählen, dass in der erhaltenen Lösung eine Konzentration an Astaxanthin-Derivat im Bereich von 100 bis 500 ppm und insbesondere im Bereich von 150 bis 500 ppm resultiert. Gemäß einer anderen Ausführungsform der Erfindung wird man die relativen Mengen an Suspension und essbarem Öl so wählen, dass eine Konzentration an Astaxanthin-Derivat im Bereich von 500 ppm bis 2,0 Gew.-%, häufig im Bereich von 500 ppm bis 1,8 Gew.-%, insbesondere im Bereich von 500 ppm bis 1,5 Gew.-% resultiert. Typischerweise liegt das Volumenverhältnis von Suspension zu dem in Schritt 2 eingesetzten Öl im Bereich von 1:200 bis 1:1 und speziell im Bereich von 1:100 bis 1:10.

Das essbare Öl, in welches die ölige Suspension des Astaxanthin-Derivats eingearbeitet wird, kann identisch mit dem zur Herstellung der Suspension verwendeten Öl sein oder eine davon verschiedene Zusammensetzung aufweisen. Vorzugsweise umfasst das zum Einarbeiten vorgesehene essbare Öl zu wenigstens 70 Gew.-%, insbesondere zu wenigstens 80 Gew.-% und speziell zu wenigstens 90 Gew.-% wenigstens ein unter Sojabohnen-Öl, Palmöl, Palmkernöl, Sonnenblumenöl, Distelöl, Maiskeimöl, Olivenöl, Leinsamenöl, Rapsöl, Reisöl, Kokosöl, Erdnussöl, PUFA-Ölen, MCT-Ölen, Fischölen sowie Mischungen dieser Öle ausgewähltes Öl. In einer besonders bevorzugten Ausführungsform der Erfindung erfasst das in Schritt ii) eingesetzte essbare Öl zu wenigstens 50 Gew.-%, insbesondere zu wenigstens 80 Gew.-%, bezogen auf die Gesamtmenge der in den Schritten i) und ii) eingesetzten essbaren Öle wenigstens ein unter Palmkernöl und MCT-Ölen ausgewähltes essbares Öl. In einer anderen bevorzugten Ausführungsform der Erfindung umfasst das in Schritt ii) eingesetzte essbare Öl zu wenigstens 50 Gew.-% und insbesondere zu wenigstens 80 Gew.-%, bezogen auf die Gesamtmenge des in den Schritten i) und ii) eingesetzten essbaren Öls wenigstens ein unter Maisöl, Sonnenblumenöl, Sojabohnenöl und Fischöl ausgewähltes essbares Öl. Das essbare Öl kann öllösliche Antioxidantien enthalten, beispielsweise Tocopherole wie α-Tocopherol, α-Tocopherolpalmitat, α-Tocopherolacetat, tert.-Butylhydroxytoluol, tert.-Butylhydroxytoluol, tert.-Butylhydroxyanisol, Ascorbylpalmitat und Ethoxyquin. Die Menge an Antioxidantien kann bis zu 20 Gew.-%, bezogen auf das Öl betragen, z. B. 0,1 bis 20 Gew.-%. Daneben kann essbare Öl, in welches die Suspension eingearbeitet wird, auch geringe Mengen an Wasser, vorzugsweise jedoch nicht mehr als 10 Gew.-%, z. B. 0,1 bis 10 Gew.-%, häufig 0,5 bis 8 Gew.-% enthalten. In einer speziellen Ausführungsform der Erfindung enthält das essbare Öl nicht mehr als 0,5 Gew.-%, an Wasser.

Das Einarbeiten der Suspension in das essbare Öl kann durch Vermischen der Suspension mit dem Öl, vorzugsweise bei erhöhter Temperatur, in üblicher Weise erfolgen. Das Mischen kann kontinuierlich oder diskontinuierlich durchgeführt werden.

Beispielsweise kann man so vorgehen, dass man ein essbares Öl in einem geeigneten Gefäß vorlegt, gegebenenfalls auf die gewünschte Temperatur vorwärmt und hierzu die Suspension des Astaxanthin-Derivats unter Durchmischen zugibt, wobei die Zugabe in einer Portion, in mehreren Portionen oder kontinuierlich erfolgen kann. Die zugegebene Suspension des Astaxanthin-Derivats kann auf die gewünschte Temperatur vorgewärmt werden, was jedoch grundsätzlich nicht erforderlich ist. Von Vorteil ist es, wenn zum Zeitpunkt des Durchmischens die Mischung eine erhöhte Temperatur, z. B. wenigstens 25 °C, insbesondere wenigstens 30 °C, z. B. eine Temperatur im Bereich von 25 bis < 100 °C, häufig im Bereich von 30 bis 90 °C, insbesondere im Bereich von 30 bis 80 °C und speziell im Bereich von 30 bis 70 °C aufweist.

Das Einarbeiten der Suspension des Astaxanthin-Derivats in das essbare Öl kann auch kontinuierlich durch sog. Inline-Mischer, d. h. kontinuierlich arbeitende Mischvorrichtungen, z. B. statische Mischer oder dynamische Mischer erfolgen. Beispiele für geeignete statische Mischer sind Mischkammern mit oder ohne Einbauten, Mischrohre mit oder ohne Mischdüsen, Jet-Mischer und der gleichen. Beispiele für dynamische Mischer sind Rotor-Stator-Mischer, Mischkammern mit Rührwerken und dergleichen. Gegebenenfalls schließt sich der Mischvorrichtung noch eine Verweilzone an, die gegebenenfalls temperiert werden kann, um das Lösen des Astaxanthin-Derivats zu vervollständigen.

Die zum Erreichen des Lösens erforderliche Mischdauer hängt naturgemäß von der Temperatur, dem gewählten Öl und den apparativen Gegebenheiten ab und liegt typischerweise im Bereich von 10 sec. bis 10 min., wobei auch kürzere Mischzeiten z. B. 5 sec. ebenso möglich sind wie längere Mischzeiten. Der Fachmann kann die erforderliche Mischzeit durch Routineexperimente ermitteln.

Im Anschluss an das Mischen kann man die erhaltene ölige Lösung, sofern erforderlich abkühlen, z. B. mittels geeigneter Wärmetauscher oder durch Verdünnen mit einem kalten essbaren Öl, das zu dem zum Lösen in Schritt ii) erhaltenen Öl gleich oder verschieden sein kann.

Auf diese Weise erhält man stabile Lösungen von Astaxanthin-Derivaten in essbaren Ölen. Diese Lösungen zeichnen sich dadurch aus, dass das in ihnen enthaltene Astaxanthin-Derivat einen hohen Anteil an all-trans-Isomeren aufweist, der in der Regel oberhalb 80 %, insbesondere oberhalb 90 % liegt. Derartige Zusammensetzungen sind neu und ebenfalls Gegenstand der vorliegenden Erfindung.

Dementsprechend betrifft die vorliegende Erfindung auch ölige Zubereitung eines Astaxanthin-Derivats, bestehend aus:
a) 0,01 bis 2,0 Gew.-%, häufig 0,01 bis 1,8 Gew.-%, insbesondere 0,015 bis 1,5 Gew.-%, bezogen auf das Gesamtgewicht der öligen Zubereitung, wenigstens eines Astaxanthin-Derivats, das zu wenigstens 70 Gew.-%, häufig zu wenigstens 80 Gew.-%, vorzugsweise zu wenigstens 90 Gew.-%, insbesondere zu wenigstens 95 Gew.-%, speziell zu wenigstens 99 Gew.-%, bezogen auf die Gesamtmenge an Astaxanthin-Derivat in der Zubereitung, aus einem Astaxanthin-Derivat der Formel I besteht,
b) einem essbaren Öl oder einer Mischung essbarer Öle, und
c) gegebenenfalls einen oder mehrere Oxidationsstabilisatoren,
worin das Astaxanthin-Derivat in dem essbaren Öl zu wenigstens 90 %, insbesondere wenigstens 95 % und speziell wenigstens 99 % in gelöster Form vorliegt und zu mehr als 80 %, insbesondere zu wenigstens 90 % eine all-trans-Konfiguration aufweist.

Bezüglich bevorzugter essbarer Öle gilt das zuvor gesagte. Die Menge an Oxidationsstabilisatoren in der Zubereitung liegt, sofern erwünscht, im Bereich von 1 bis 30 Gew.-%, bezogen auf das in der Zubereitung enthaltene Astaxanthin-Derivat. Die erfindungsgemäße ölige Zubereitung kann Antioxidantien enthalten, beispielsweise öllösliche Antioxidantien, z. B. Tocopherole wie α-Tocopherol, α-Tocopherolpalmitat, α-Tocopherolacetat, tert.-Butylhydroxytoluol, tert.-Butylhydroxytoluol, tert.-Butylhydroxyanisol, Ascorbylpalmitat und Ethoxyquin und in Ölen nicht oder schlecht lösliche Antioxidantien wie die vorgenannten Salze der Ascorbinsäure. Die Menge an öllöslichen Antioxidantien kann bis zu 20 Gew.-%, bezogen auf das Öl betragen, z. B. 0,1 bis 20 Gew.-%. Im Falle nicht oder schlecht löslicher Antioxidantien beträgt ihre Menge, sofern vorhanden, typischerweise nicht mehr als 1 Gewichtsteil, z. B. 0,01 bis 1 Gewichtsteil, bezogen auf 1 Gewichtsteil des Astaxanthin-Derivats.

Es versteht sich von selber, dass die erfindungsgemäße ölige Zubereitung geringe Mengen an Wasser aus dem zu ihrer Herstellung eingesetzten Öl enthalten kann, das dem essbaren Öl zugerechnet wird. Vorzugsweise jedoch beträft der Wasseranteil nicht mehr als 10 Gew.-%, z. B. 0,1 bis 10 Gew.%, häufig 0,5 bis 8 Gew.-%, und in einer speziellen Ausführungsform der Erfindung nicht mehr als 0,5 Gew.-%, bezogen auf das in der Zusammensetzung enthaltene Öl.

Die erfindungsgemäß erhältlichen öligen Zubereitungen bzw. Lösungen von Astaxanthin-Derivaten sind lagerstabil und können vor ihrer weiteren Verwendung über längere Zeiträume aufbewahrt werden, ohne dass es in nennenswerten Umfang zu einem Aktivitätsverlust, z. B. durch Isomerisierung und/oder oxidativen Abbau, oder zu einer Kristallisation des Astaxanthin-Derivats kommt. Das erfindungsgemäße Verfahren kann aber in die Prozesse zur Weiterverarbeitung der öligen Zubereitungen integriert werden. In diesem Fall wird man vorzugsweise die in Schritt i) erhaltene Suspension kontinuierlich in das essbare Öl einarbeiten.

Die erfindungsgemäßen bzw. nach dem erfindungsgemäßen Verfahren erhältlichen öligen Zubereitungen bzw. Lösungen von Astaxanthin-Derivaten eignen sich in vorteilhafter Weise als Zusatz zu Tierfuttermitteln, Nahrungsmitteln für die Humanernährung, Nahrungsergänzungsmitteln, pharmazeutischen Mitteln oder kosmetischen Mitteln. Bevorzugt lassen sich die öligen Zubereitungen bzw. Lösungen als Futtermittelzusatz in der Tierernährung einsetzen, beispielsweise durch Einmischen in Futtermittelpellets bei der Extrusion oder durch Auftragen bzw. Aufsprühen auf Futtermittelpellets. Die Anwendung als Futtermittelzusatz erfolgt insbesondere durch direktes Aufsprühen der erfindungsgemäßen Formulierungen, beispielsweise als sogenannte "post-pelleting application". Vorzugsweise belädt man die Futtermittelpellets mit den Formulierungen unter vermindertem Druck.

Dementsprechend betrifft die vorliegende Erfindung auch Mittel in Form eines Tierfuttermittels, Nahrungsmittels für die Humanernährung, Nahrungsergänzungsmittels, pharmazeutischen Mittels oder kosmetischen Mittels, welche eine erfindungsgemäße ölige Zubereitung umfassen. Diese Mittel enthalten neben den für diese Mittel üblichen Bestandteile das wenigstens eine essbare Öl und ein Astaxanthin-Derivat, das zu wenigstens 70 Gew.-%, häufig zu wenigstens 80 Gew.-%, vorzugsweise zu wenigstens 90 Gew.-%, insbesondere zu wenigstens 95 Gew.-%, speziell zu wenigstens 99 Gew.-%, bezogen auf die Gesamtmenge an Astaxanthin-Derivat in dem Mittel, aus einem Astaxanthin-Derivat der Formel I besteht, und wobei das in dem Mittel enthaltene Astaxanthin-Derivat zu mehr als 80 %, insbesondere zu wenigstens 90 % eine all-trans-Konfiguration aufweist.

Bevorzugte Ausführungsformen betreffen Tierfuttermittel insbesondere Fischfuttermittel, die die erfindungsgemäße ölige Zubereitung umfassen. Derartige Mittel enthaltend das in der öligen Zubereitung enthaltene Astaxanthin-Derivat typischerweise in einer Menge von 10 bis 100 ppm, bezogen auf das Gesamtgewicht des Mittels, wobei das Astaxanthin-Derivat in der Regel zu mehr als 80 %, insbesondere zu wenigstens 90 % eine all-trans-Konfiguration aufweist.

Typische Bestandteile in Futtermitteln sind Kohlehydrat-Quellen, insbesondere Getreidemehle wie Weizen- oder Maismehl, Sojabohnenmehl, aber auch Zucker und Zuckeralkohole, weiterhin proteinhaltige Bestandteile wie Sojakonzentrat, Fischmehl, Glutene wie Mais- oder Weizengluten, Öle und Fette, z. B. die zuvor genannten essbaren Öle, aber auch andere essbare Fette pflanzlichen oder tierischen Ursprungs weiterhin Nutrazeutika wie freie Aminosäuren, deren Salze, Vitamine und Spurenelemente, sowie gegebenenfalls Verarbeitungshilfsmittel, z. B. Gleitmittel, Antiblockmittel, inerte Füllstoffe und dergleichen und gegebenenfalls Konservierungsmittel. Typische Fischfutterzusammensetzungen enthalten z. B. Getreidemehl in einer Menge von beispielsweise 3 bis 20 Gew.-%, Gluten, z. B. in einer Menge von 1 bis 30 Gew.-%, ein oder mehrere Proteinquellen, z.B. Sojakonzentrat und/oder Fischmehl, z. B. in einer Gesamtmenge von 10 bis 50 Gew.-%, Fette und/oder Öle in einer Menge von beispielsweise 10 bis 50 Gew.-%, gegebenenfalls ein oder mehrere Vitamine in einer Gesamtmenge von beispielsweise 0,1 bis 2 Gew.-% und gegebenenfalls Aminosäuren in einer Menge von beispielsweise 0,1 bis 5 Gew.-%, jeweils bezogen auf die Gesamtmenge der Futtermittelbestandteile.

Eine spezielle Ausführungsform der Erfindung betrifft Futtermittelpellets, speziell Futtermittelpellets für Fischfutter, die mit den erfindungsgemäßen öligen Zubereitungen beladen sind. Derartige Pellets enthaltend das in der öligen Zubereitung enthaltene Astaxanthin-Derivat typischerweise in einer Menge von 10 bis 100 ppm, bezogen auf das Gesamtgewicht des Futtermittels, wobei das Astaxanthin-Derivat in der Regel zu mehr als 80 %, insbesondere zu wenigstens 90 % eine all-trans-Konfiguration aufweist.

Die Herstellung solcher Pellets erfolgt in der Regel durch Besprühen konventioneller Pellets mit einer erfindungsgemäßen öligen Zusammensetzung, vorzugsweise unter vermindertem Druck, wobei das Besprühen kontinuierlich oder vorzugsweise diskontinuierlich erfolgen kann. Beispielsweise kann man die konventionellen Pellets in einem Geeigneten Gefäß vorlegen, dass Gefäß evakuieren und dann Öl unter Durchmischen der Pellets aufsprühen und anschließend belüften. Auf diese Weise erreicht man ein gleichmäßiges Eindringen der erfindungsgemäßen öligen Zusammensetzung in die Pellets. Gegebenfalls kann man erneut Vakuum anlegen und erneut eine erfindungsgemäße ölige Zusammensetzung oder ein essbares Öl in der zuvor beschriebenen Weise aufsprühen. Auf diese Weise erhält man Pellets, die im Kern das Öl enthalten.

Die folgenden Beispiele dienen der weiteren Erläuterung der Erfindung.

### I. Analytik:

Die Bestimmung der Partikelgrößen erfolgte mittels Fraunhoferbeugung unter Verwendung eines Malvern Mastersizer S bei 22 °C. Die durch Vermahlen erhaltenen Suspensionen des Astaxanthin-Derivats wurden vor der Messung mit dem zum Vermahlen verwendeten Öl auf eine Konzentration von 0,05 Gew.-% verdünnt. Angegeben ist der volumenmittlere Teilchendurchmesser D_{4,3}.

Zur Bestimmung des absoluten Gehalts an Astaxanthin-Derivat in den erfindungsgemäß erhältlichen Suspensionen löste man die Suspension in Aceton und bestimmte anschließend mittels UVNIS-Spektroskopie (200-800 nm) die Extinktion der Lösung bei 478 nm bzw. 476 nm (Astaxanthin).

Zur Bestimmung des relativen Gehalts an gelöstem Astaxanthin-Derivat in den erfindungsgemäßen Lösungen, bezogen auf die eingesetzte Menge an Astaxanthin-Derivat, filtrierte man die Proben über einen Filter mit einer Porenweite von 0,2 µm und bestimmte anschließend mittels UVNIS-Spektroskopie (200-800 nm) die Extinktion der Lösung bei 478 nm bzw. 476 nm (Astaxanthin).

### II. Einsatzmaterialien:

Für die Versuche wurden die folgenden Astaxanthin-Derivate eingesetzt:

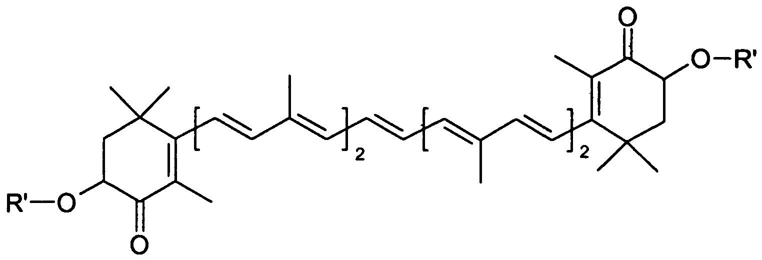

| R' | Bezeichnung | Abkürzung | Referenz |
|---|---|---|---|
| H | Astaxanthin | Ax | CAS 472-61-7 |
| C(=O)CH₂CH₂C(=O)OCH₃ | Astaxanthin-Dimethyldisuccinat | Ax-DMDS | WO 03/066583 |
| C(=O)CH₃ | Astaxanthin-Diacetat | Ax-DA | * |
| C(=O)CH₂CH₂C(=O)OH | Astaxanthin-Disuccinat | Ax-DS | WO 03/066583 |
| C(=O)(CH₂)₁₄CH₃ | Astaxanthin-Dipalmitat | Ax-DP | * |

| | | | |
|---|---|---|---|
| *hergestellt durch Veresterung von Astaxanthin | | | |

### Extinktionskoeffizienten:

| Abkürzung | E 1/1 * |
|---|---|
| Ax | 2040 |
| Ax-DMDS | 1250 |
| Ax-DA | 1793 |
| Ax-DS | 1150 |
| Ax-DP | 1162 |

| | |
|---|---|
| * Extinktion einer 1 gew.-%igen Lösung in einer 1 cm Küvette | |

Astaxanthin und Derivate Ax-DMDS, Ax-DS, Ax-DA und Ax-DP wiesen eine Reinheit > 95 % auf.

Sonnenblumenöl: handelsübliches Produkt mit Lebensmittelqualität der Fa. Gustav Heess GmbH; Wassergehalt < 0,1 %; Fettsäureverteilung: 4-9 % Palmitinsäure, 1-7 % Stearinsäure, 14-40 % Ölsäure, 48-70 % Linolsäure.

Palmkernöl: handelsübliches Raffinat mit Lebensmittelqualität; Wassergehalt < 0,1 %; Gehalt an C12/C14-Carbonsäuretriglycieriden > 60 %.

MCT-Öl: Delios® MCT-Öl der Fa. Cognis GmbH; Wassergehalt < 0,1 %, Gehalt an C8/C10-Fettsäuretriglyceriden > 95 %.

PUFA-Öl: handelsübliches Produkt mit Lebensmittelqualität der Fa. Napro-Pharma AS, Norwegen; Eicosahexaensäure > 250 mg/g, Eicosapentaesäure < 57 mg/g, bestimmt nach European Pharmacoepia 2.4.29.

### III. Herstellung der Astaxanthin-Lösungen:

### 1. Allgemeine Vorschrift für das Vermahlen von Ax, Ax-DMDS, Ax-DA und Ax-DS

8 g bzw. 12 g Astaxanthin bzw. das Astaxanthin-Derivat wurden mit 392 g bzw. 388 g Sonnenblumenöl vermischt so dass man eine 2 gew.-%ige bzw. 3 gew.-%ige Primärsuspension erhielt. Diese Primärsuspension wurde anschließend mit 4 Passagen bei Temperaturen im Bereich von 42-48°C (Astaxanthin) bzw. 50-55 °C (Astaxanthin-Derivate), mit einem Druck von 0,2 bar, einer Durchflussrate von 60 bis 70 g/min und einer Mahldauer pro Passage von etwa 5 min. in einer Kugelmühle (Dynomill: Reibspalt 0,05 mm, 4180 U/min, 470 ml, gefüllt mit ZrO₂-stablisierten Kugeln mit einem Durchmesser von 0,2 bis 0,4 mm) vermahlen. Nach jedem Mahlgang wurde der Teilchendurchmesser der suspendierten Partikel bestimmt. Die Ergebnisse sind in den folgenden Tabellen 1 bis 4 zusammengestellt.

### 1.1 3 %ige Suspension von Ax-DMDS in Sonnenblumenöl, 3 %ige Suspension

**Tabelle 1: Mahlung von Ax-DMDS in Sonnenblumenöl (Mahlung bei 50 °C) - erfindungsgemäß**

| Probe | Gehalt | D_{4.3} (µm) |
|---|---|---|
| Ausgang | 3,0% | 130,96 |
| 1. Passage | | 2,79 |
| 2.Passage | | 2,30 |
| 3. Passage | | 1,93 |
| 4. Passage | 2,95% | 1,66 |

### 1.2 3 %ige Suspension von Ax in Sonnenblumenöl

**Tabelle 2: Mahlung von Ax in Sonnenblumenöl (Mahlung bei 42-48 °C) - Vergleich**

| Probe | Gehalt | D_{4.3} (µm) |
|---|---|---|
| Ausgang | 3,0 % | 29,75 |
| 1. Passage | | 2,39 |
| 2.Passage | | 1,74 |
| 3. Passage | | 1,50 |
| 4. Passage | 2,86% | 1,14 |

### 1.3 2 %ige Suspension von Ax-DS in Sonnenblumenöl

**Tabelle 3: Mahlung von Ax-DS in Sonnenblumenöl (Mahlung bei 50-55 °C) - Vergleich**

| Probe | Gehalt | D_{4.3} (µm) |
|---|---|---|
| Ausgang | 2,0% | 48,39 |
| 1. Passage | | Nicht gemessen |
| 2.Passage | | Nicht gemessen |
| 3. Passage | | Nicht gemessen |
| 4. Passage | 0,52% | 17,07 |

Ca. 75 % des Wirkstoffes bleibt in der Mühle hängen. Größere Mengen an Ax-DS würden zu einem Zusetzen der Mühle führen.

### 1.4 2 %ige Suspension von Ax-DA in Sonnenblumenöl

**Tabelle 4: Mahlung von Ax-DA in Sonnenblumenöl (Mahlung bei 50-55 °C) - Vergleich**

| Probe | Gehalt | D_{4.3} (µm) |
|---|---|---|
| Ausgang | 2,0 % | Nicht gemessen |
| 1. Passage | | 2,64 |
| 2.Passage | | Nicht gemessen |
| 3. Passage | | Nicht gemessen |
| 4. Passage | 1,96% | 0,82 |

### 2. Vorschrift für das Vermahlen von Ax-DP - Vergleich

Da die Ax-DP Kristalle im Vergleich zu Ax-DMDS, Ax, Ax-DA und Ax-DS weicher sind, wurde von einer Mahlung mittels einer Kugel-Mühle nicht angefangen, da die Mühle sich zusetzen würde. Durch Zerkleinerung einer 10%igen Suspension von Ax-DP in Sonnenblumenöl mit einem Mörser wurde eine volumenmittlere Partikelgröße von D_{4,3} = 5,2 µm erreicht.

### 3. Allgemeine Vorschrift für das Lösen der Suspensionen von Ax, Ax-DMDS, Ax-DA, Ax-DP bzw. Ax-DS

In einem Gefäß mit Rührer wurde die jeweils angegebene Menge an Öl vorgelegt und auf die gewünschte Temperatur erwärmt. Hierzu gab man unter Rühren die angegebene Menge an Suspension. Nach bestimmten Zeiten wurden Proben genommen, über einen Filter mit einer Porenweite von 0,2 µm filtriert und anschließend der Gehalt an Astaxanthin-Derivat bzw. Astaxanthin mittels UV/VIS-Spektroskopie bestimmt. Die Ergebnisse sind in den folgenden Tabellen 5 bis 15 zusammengestellt.

### 3.1 Lösen von Ax-DMDS in Sonnenblumenöl, 30 °C, 370 ppm

1,2 g der Ax-DMDS-Suspension aus dem 4. Mahlgang (D_{4.3} = 1,66 µm) wurden in 98,8 g Sonnenblumenöl, das auf 30 °C erwärmt war, eingearbeitet. Dies entspricht einer maximalen Konzentration von 370 ppm Ax-DMDS. Die Ergebnisse sind in Tabelle 5 angegeben.

**Tabelle 5:**

| Versuchsparameter | Zeit [sec]¹⁾ | Ax-DMDS [ppm]²⁾ | Ax-DMDS [%]³⁾ |
|---|---|---|---|
| Sonnenblumenöl | 0 | 0 | 0 |
| 30 °C | 10 | 144 | 39 |
| 370 ppm | 20 | 179 | 54 |
| | 60 | 214 | 61 |
| | 300 | 255 | 72 |
| | 3600 | 270 | 75 |

| | | | |
|---|---|---|---|
| 1) Zeitpunkt der Probennahme 2) Konzentration an gelöstem Ax-DMDS 3) Anteil an gelöstem Ax-DMDS, bezogen auf das in der Suspension enthaltene Ax-DMDS | | | |

### 3.2 Lösen von Ax-DMDS in Sonnenblumenöl, 30 °C, 250 ppm

0,85 g der Ax-DMDS-Suspension aus dem 4. Mahlgang (D_{4.3} = 1,66 µm) wurden in 99,15 g Sonnenblumenöl, das auf 30 °C erwärmt war, eingearbeitet. Dies entspricht einer maximalen Konzentration von 250 ppm Ax-DMDS.

### 3.3 Lösen von Ax-DMDS in Sonnenblumenöl, 70 °C, 365 ppm

1,2 g der in 1.1 hergestellten Ax-DMDS-Suspension aus dem 4. Mahlgang (D_{4.3} = 1,66 µm) wurden in 98,8 g Sonnenblumenöl, das auf 70 °C erwärmt war, eingearbeitet. Dies entspricht einer maximalen Konzentration von 365 ppm Ax-DMDS. Die Ergebnisse sind in Tabelle 6 angegeben.

**Tabelle 6:**

| Versuchsparameter | Zeit [sec]¹⁾ | Ax-DMDS [ppm]²⁾ | Ax-DMDS [%]³⁾ |
|---|---|---|---|
| Sonnenblumenöl | 0 | 0 | 0 |
| 70 °C | 13 | 308 | 84 |
| 365 ppm | 26 | 323 | 88 |
| | 39 | 345 | 95 |
| | 52 | 352 | 96 |
| | 65 | 352 | 96 |
| | 78 | 359 | 98 |
| | 132 | 364 | 100 |
| | 144 | 365 | 100 |
| | 156 | 363 | 100 |
| | 180 | 363 | 100 |

| | | | |
|---|---|---|---|
| 1), 2), 3): Legende siehe Tabelle 5 | | | |

Das UV-VIS Spektrum bleibt über einem Zeitraum von 3 h unverändert, d. h. bei 70 °C in Sonneblumenöl ist die Isomerisierung bzw. der Abbau von Ax-DMDS über einen Zeitraum von 3 Std. vernachlässigbar.

### 3.4 Lösen von Ax-DMDS in Sonnenblumenöl, 70 °C, 330 ppm

1,1 g der in 1.1 hergestellten Ax-DMDS-Suspension aus dem 1. Mahlgang (D_{4.3} = 2,79 µm) wurden in 98,9 g Sonnenblumenöl, das auf 70 °C erwärmt war, eingearbeitet. Dies entspricht einer maximalen Konzentration von 330 ppm Ax-DMDS. Die Ergebnisse sind in Tabelle 7 angegeben.

**Tabelle 7**

| Versuchsparameter | Zeit [sec]¹⁾ | Ax-DMDS [ppm]²⁾ | Ax-DMDS [%]³⁾ |
|---|---|---|---|
| Sonnenblumenöl | 0 | 0 | 0 |
| 70 °C | 20 | 271 | 81 |
| 330 ppm | 40 | 291 | 87 |
| | 80 | 307 | 92 |
| | 180 | 329 | 99 |
| | 240 | 333 | 100 |

| | | | |
|---|---|---|---|
| 1), 2), 3): Legende siehe Tabelle 5 | | | |

Die Versuche 3.3 und 3.4 zeigen, dass die Partikelgröße einen Einfluss auf die Lösungskinetik hat.

Nicht vermahlenes Ax-DMDS mit einer Partikelgröße von etwa 130 µm löste sich nach 1 h bei 70 °C in Sonnenblumenöl nur zu 70 %.

### 3.5 Lösen von Ax-DMDS in PUFA-Öl, 70 °C, 350 ppm

1,1 g der in 1.1 hergestellten Ax-DMDS-Suspension aus dem 1. Mahlgang (D_{4.3} = 2,79 µm) wurden in 98,7 g PUFA-Öl, das auf 70 °C erwärmt war, eingearbeitet. Dies entspricht einer maximalen Konzentration von 350 ppm Ax-DMDS.

### 3.6 Lösen von Ax-DMDS in PUFA-Öl, 30 °C, 385 ppm

1,3 g der in 1.1 hergestellten Ax-DMDS-Suspension aus dem 4. Mahlgang (D_{4.3} = 1,66 µm) wurden in 98,9 g PUFA-Öl, das auf 30 °C erwärmt war, eingearbeitet. Dies entspricht einer maximalen Konzentration von 385 ppm Ax-DMDS. Die Ergebnisse sind in Tabelle 8 angegeben.

**Tabelle 8**

| Versuchsparameter | Zeit [sec]¹⁾ | Ax-DMDS [ppm]²⁾ | Ax-DMDS [%]³⁾ |
|---|---|---|---|
| PUFA Öl | 0 | 0 | 0 |
| 30 °C | 10 | 150 | 39 |
| 385 ppm | 20 | 179 | 46 |
| | 60 | 214 | 55 |
| | 300 | 255 | 66 |
| | 3600 | 270 | 70 |

| | | | |
|---|---|---|---|
| 1), 2), 3): Legende siehe Tabelle 5 | | | |

### 3.7 Lösen von Ax-DMDS in MCT-Öl, 30 °C, 380 ppm

1,3 g der in 1.1 hergestellten Ax-DMDS-Suspension aus dem 4. Mahlgang (D_{4.3} = 1,66 µm) wurden in 98,7 g MCT-Öl, das auf 30 °C erwärmt war, eingearbeitet. Dies entspricht einer maximalen Konzentration von 380 ppm Ax-DMDS. Die Ergebnisse sind in Tabelle 9 angegeben.

**Tabelle 9**

| Versuchsparameter | Zeit [sec]¹⁾ | Ax-DMDS [ppm]²⁾ | Ax-DMDS [%]³⁾ |
|---|---|---|---|
| MCT Öl | 0 | 0 | 0 |
| 30 °C | 10 | 329 | 87 |
| 380 ppm | 60 | 359 | 94 |
| | 90 | 377 | 99 |
| | 300 | 380 | 100 |

| | | | |
|---|---|---|---|
| 1), 2), 3): Legende siehe Tabelle 5 | | | |

### 3.8 Lösen von Ax-DMDS in MCT-Öl, 70 °C, 360 ppm

1,2 g der in 1.1 hergestellten Ax-DMDS-Suspension aus dem 4. Mahlgang (D_{4.3} = 1,66 µm) wurden in 98,8 g MCT-Öl, das auf 70 °C erwärmt war, eingearbeitet. Dies entspricht einer maximalen Konzentration von 360 ppm Ax-DMDS. Die Ergebnisse sind in Tabelle 10 angegeben.

**Tabelle 10**

| Versuchsparameter | Zeit [sec]¹⁾ | Ax-DMDS [ppm]²⁾ | Ax-DMDS [%]³⁾ |
|---|---|---|---|
| MCT Öl | 0 | 0 | 0 |
| 70 °C | 10 | 353 | 97 |
| ca. 360 ppm | 20 | 356 | 98 |
| | 30 | 362 | 100 |
| | 40 | 362 | 100 |
| | 60 | 361 | 100 |
| | 180 | 363 | 100 |

| | | | |
|---|---|---|---|
| 1), 2), 3): Legende siehe Tabelle 5 | | | |

### 3.9 Lösen von Ax-DMDS in Palmkernöl, 70 °C, 360 ppm

1,2 g der in 1.1 hergestellten Ax-DMDS-Suspension aus dem 4. Mahlgang (D_{4.3} = 1,66 µm) wurden in 98,8 g Palmkernöl, das auf 70 °C erwärmt war, eingearbeitet. Dies entspricht einer maximalen Konzentration von 360 ppm Ax-DMDS.

### 3.10 Lösen von Ax-DMDS in Palmkernöl, 30 °C, 360 ppm

1,2 g der in 1.1 hergestellten Ax-DMDS-Suspension aus dem 4. Mahlgang (D_{4.3} = 1,66 µm) wurden in 98,8 g Palmkernöl, das auf 30 °C erwärmt war, eingearbeitet. Dies entspricht einer maximalen Konzentration von 360 ppm Ax-DMDS.

### 3.11 Lösen von Ax in Sonnenblumenöl, 350 ppm (Vergleich)

1,25 g der in 1.2 hergestellten Ax -Suspension aus dem 4. Mahlgang (D_{4.3} = 1,14 µm) wurden in 98,75 g Sonnenblumenöl, das auf 70 °C erwärmt war, eingearbeitet. Dies entspricht einer maximalen Konzentration von 350 ppm Ax. Die Ergebnisse sind in Tabelle 11 zusammengestellt.

**Tabelle 11**

| Versuchsparameter | Zeit [sec]¹⁾ | Ax [ppm]²⁾ | Ax [%]³⁾ |
|---|---|---|---|
| Sonnenblumenöl | 0 | 0 | 0 |
| 70 °C | 10 | 68 | 19 |
| ca. 350 ppm | 20 | 68 | 19 |
| | 60 | 72 | 21 |
| | 300 | 73 | 21 |
| | 3600 | 93 | 27 |

| | | | |
|---|---|---|---|
| 1), 2), 3): Legende siehe Tabelle 5 | | | |

### 3.12 Lösen von Ax-DS in Sonnenblumenöl, 350 ppm (Vergleich)

1,2 g der in 1.3 hergestellten Ax-DS-Suspension aus dem 4. Mahlgang (D_{4.3} = 17,1 µm) wurden in 98,8 g Sonnenblumenöl, das auf 70 °C erwärmt war, eingearbeitet. Dies entspricht einer maximalen Konzentration von 350 ppm Ax-DS. Die Ergebnisse sind in Tabelle 12 zusammengestellt.

**Tabelle 12**

| Versuchsparameter | Zeit [sec]¹⁾ | Ax-DS [ppm]²⁾ | Ax-DS [%]³⁾ |
|---|---|---|---|
| Sonnenblumenöl | 0 | 0 | 0 |
| 70 °C | 10 | 107 | 31 |
| ca. 350 ppm | 60 | 115 | 33 |
| | 3600 | 189 | 54 |

| | | | |
|---|---|---|---|
| 1), 2), 3): Legende siehe Tabelle 5 | | | |

### 3.13 Lösen von Ax-DA in Sonnenblumenöl, 350 ppm (Vergleich)

1,2 g der in 1.4 hergestellten Ax-DA-Suspension aus dem 4. Mahlgang (D_{4.3} = 0,82 µm) wurden in 98,8 g Sonnenblumenöl, das auf 70 °C erwärmt war, eingearbeitet. Dies entspricht einer maximalen Konzentration von 350 ppm Ax-DA. Die Ergebnisse sind in Tabelle 13 zusammengestellt.

**Tabelle 13**

| Versuchsparameter | Zeit [sec]¹⁾ | Ax-DA [ppm]²⁾ | Ax-DA [%]³⁾ |
|---|---|---|---|
| Sonnenblumenöl | 0 | 0 | 0 |
| 70 °C | 10 | 111 | 32 |
| ca. 350 ppm | 20 | 115 | 333 |
| | 60 | 139 | 40 |
| | 300 | 147 | 42 |
| | 3600 | 145 | 41 |

| | | | |
|---|---|---|---|
| 1), 2), 3): Legende siehe Tabelle 5 | | | |

### 3.14 Lösen von Ax-DA in MCT-Öl, 350 ppm (Vergleich)

1,2 g der in 1.4 hergestellten Ax-DA-Suspension aus dem 4. Mahlgang (D_{4.3} = 0,82 µm) wurden in 98,8 g MCT-Öl, das auf 70 °C erwärmt war, eingearbeitet. Dies entspricht einer maximalen Konzentration von 350 ppm Ax-DA. Die Ergebnisse sind in Tabelle 14 zusammengestellt.

**Tabelle 14**

| Versuchsparameter | Zeit [sec]¹⁾ | Ax-DA [ppm]²⁾ | Ax-DA [%]³⁾ |
|---|---|---|---|
| MCT Öl | 0 | 0 | 0 |
| 70 °C | 10 | 280 | 80 |
| ca. 350 ppm | 20 | 290 | 83 |
| | 60 | 296 | 85 |
| | 300 | 303 | 87 |
| | 3600 | 297 | 85 |

| | | | |
|---|---|---|---|
| 1), 2), 3): Legende siehe Tabelle 5 | | | |

### 3.15 Lösen von Ax-DP in Sonnenblumenöl, 350 ppm (Vergleich)

1,2 g der in 2 hergestellten Ax-DP-Suspension (D_{4.3} = 5,2 µm) wurden in 98,8 g Sonnenblumenöl, das auf 70 °C erwärmt war, eingearbeitet. Dies entspricht einer maximalen Konzentration von 350 ppm Ax-DP. Die Ergebnisse sind in Tabelle 15 zusammengestellt.

**Tabelle 15**

| Versuchsparameter | Zeit [sec]¹⁾ | Ax-DP [ppm]²⁾ | Ax-DP [%]³⁾ |
|---|---|---|---|
| Sonnenblumenöl | 0 | 0 | 0 |
| 70 °C | 10 | 220 | 61 |
| ca. 350 ppm | 20 | 277 | 77 |
| | 60 | 344 | 96 |
| | 300 | 350 | 100 |
| | 3600 | 350 | 100 |

| | | | |
|---|---|---|---|
| 1), 2), 3): Legende siehe Tabelle 5 | | | |

## Patentansprüche

1. Verfahren zur Herstellung einer Lösung eines Astaxanthin-Derivats in einem essbaren Öl, umfassend
i) Bereitstellen einer Suspension des Astaxanthin-Derivats in dem essbaren Öl, wobei die Partikel des Astaxanthin-Derivats in der Suspension einen volumenmittleren Teilchendurchmesser, bestimmt durch Fraunhofer-Beugung, im Bereich von 0,5 bis 50 µm aufweisen, und
ii) Einarbeiten der Suspension des Astaxanthin-Derivats in ein essbares Öl, wobei man eine Lösung des Astaxanthin-Derivats erhält.
wobei das Astaxanthin-Derivat zu wenigstens 70 Gew.-% aus wenigstens einer Verbindung der Formel I 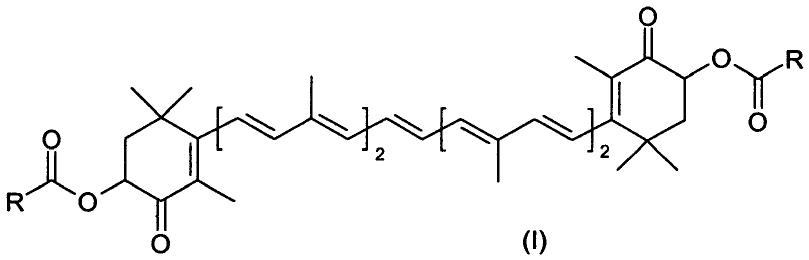 besteht, worin R für einen Rest der Formel 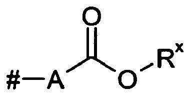 steht, worin # die Anknüpfung an die Carbonylgruppe bedeutet, A für lineares oder verzweigtes C₁-C₆-Alkylen oder für -CH=CH- steht und Rₓ für C₁-C₁₀ Alkyl steht.

2. Verfahren nach Anspruch 1, wobei der Rest R in Formel I für einen Rest der Formel 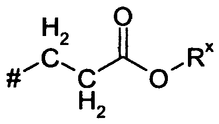 steht, worin R^{x} Methyl oder Ethyl bedeutet.

3. Verfahren nach einem der vorhergehenden Ansprüche, wobei die Suspension des Astaxanthin-Derivats in dem essbaren Öl durch Vermahlen des Astaxanthin-Derivats in dem essbaren Öl hergestellt wird.

4. Verfahren nach einem der vorhergehenden Ansprüche, wobei in Schritt i) eine Temperatur von 100 °C nicht überschritten wird.

5. Verfahren nach einem der vorhergehenden Ansprüche, wobei man in Schritt i) 1 bis 20 Gew.-% des Astaxanthin-Derivats, bezogen die Gesamtmenge an essbarem Öl und Astaxanthin-Derivat einsetzt.

6. Verfahren nach einem der vorhergehenden Ansprüche, wobei das in den Schritten i) und ii) eingesetzte essbare Öl ausgewählt ist unter Sojabohnen-Öl, Palmöl, Palmkernöl, Sonnenblumenöl, PUFA-Ölen, MCT-Ölen, Fischölen, Distelöl, Maisöl, Olivenöl, Leinsamenöl, Rapsöl, Reisöl und Mischungen dieser Öle.

7. Verfahren nach einem der vorhergehenden Ansprüche, wobei das essbare Öl in Schritt ii) eine Temperatur im Bereich von 30 bis < 100 °C aufweist.

8. Verfahren nach einem der vorhergehenden Ansprüche, wobei man in Schritt ii) die Suspension unter Rühren bei erhöhter Temperatur zu dem essbaren Öl gibt.

9. Verfahren nach einem der Ansprüche 1 bis 7, wobei man in Schritt ii) die Suspension in einem Inline-Mischer mit dem essbaren Öl bei erhöhter Temperatur vermischt.

10. Ölige Suspension eines Astaxanthin-Derivats der Formel I, bestehend aus:
a) 1 bis 20 Gew.-% wenigstens eines feinteiligen Astaxanthin-Derivats, das zu wenigstens 70 Gew.-%, bezogen auf die Gesamtmenge an Astaxanthin-Derivat in der Suspension, aus einem Astaxanthin-Derivat der Formel I gemäß Anspruch 1 oder 2 besteht,
b) 80 bis 99 Gew.-%, eines essbaren Öls oder einer Mischung essbarer Öle, und
c) gegebenenfalls einen oder mehrere Oxidationsstabilisatoren,
worin die Teilchen des Astaxanthin-Derivats in der Suspension einen volumenmittleren Teilchendurchmesser, bestimmt durch Fraunhofer-Beugung, im Bereich von 0,5 bis 50 µm aufweisen, und die Angaben in Gew.-% auf das Gesamtgewicht der öligen Suspension bezogen sind.

11. Ölige Zubereitung eines Astaxanthin-Derivats, bestehend aus:
a) 0,01 bis 2,0 Gew.-%, bezogen auf das Gesamtgewicht der öligen Zubereitung, wenigstens eines Astaxanthin-Derivats, bestehend zu wenigstens 70 Gew.-% aus dem Astaxanthin-Derivat der Formel I gemäß Anspruch 1 oder 2 besteht,
b) einem essbaren Öl oder einer Mischung essbarer Öle, und
c) gegebenenfalls einen oder mehrere Oxidationsstabilisatoren,
worin das Astaxanthin-Derivat in dem essbaren Öl zu wenigstens 90 % in gelöster Form vorliegt und zu > 80 % eine all-trans-Konfiguration aufweist.

12. Verwendung einer öligen Zubereitung gemäß Anspruch 11 als Zusatz zu Tierfuttermitteln, Nahrungsmitteln, Nahrungsergänzungsmittein, pharmazeutischen Mitteln oder kosmetischen Mitteln.

13. Mittel in Form eines Tierfuttermittels, Nahrungsmittels, Nahrungsergänzungsmittels, pharmazeutischen Mittels oder kosmetischen Mittels, umfassend eine ölige Zubereitung nach Anspruch 11.

14. Mittel nach Anspruch 13 in Form von Pellets für Tierfutter.

15. Mittel nach Anspruch 13 oder 14, enthaltend das Astaxanthin-Derivat in einer Menge von 10 bis 100 ppm, bezogen auf das Gesamtgewicht des Mittels.

## Claims

1. A process for producing a solution of an astaxanthin derivative in an edible oil which comprises
i) providing a suspension of the astaxanthin derivative in the edible oil, wherein the particles of the astaxanthin derivative in the suspension have a volume-average particle diameter determined by Fraunhofer diffraction in the range from 0.5 to 50 µm, and
ii) incorporating the suspension of the astaxanthin derivative into an edible oil, wherein a solution of the astaxanthin derivative is obtained,
wherein the astaxanthin derivative comprises at least 70% by weight of at least one compound of the formula I 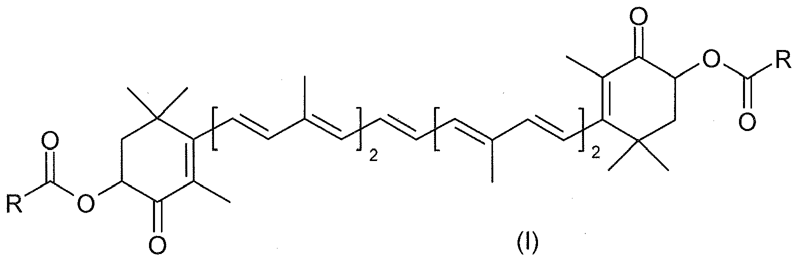 where R is a radical of the formula 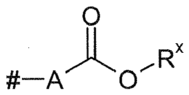 where # indicates the linkage to the carbonyl group, A is a linear or branched C₁-C₆-alkylene or -CH=CH- and R^{x} is C₁-C₁₀ alkyl.

2. The process according to claim 1, wherein the radical R in formula I is a radical of the formula 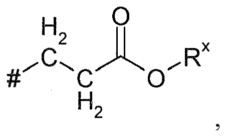 where R^{x} is methyl or ethyl.

3. The process according to one of the preceding claims, wherein the suspension of the astaxanthin derivative in the edible oil is produced by grinding the astaxanthin derivative in the edible oil.

4. The process according to one of the preceding claims, wherein, in step i), a temperature of 100°C is not exceeded.

5. The process according to one of the preceding claims, wherein, in step i), use is made of 1 to 20% by weight of the astaxanthin derivative, based on the total amount of edible oil and astaxanthin derivative.

6. The process according to one of the preceding claims, wherein the edible oil used in steps i) and ii) is selected from soybean oil, palm oil, palm kernel oil, sunflower oil, PUFA oils, MCT oils, fish oils, safflower oil, corn oil, olive oil, linseed oil, rapeseed oil, rice oil and mixtures of these oils.

7. The process according to one of the preceding claims, wherein the edible oil in step ii) has a temperature in the range from 30 to < 100°C.

8. The process according to one of the preceding claims, wherein, in step ii), the suspension is added to the edible oil at elevated temperature with stirring.

9. The process according to one of claims 1 to 7, wherein, in step ii), the suspension is mixed with the edible oil at elevated temperature in an inline mixer.

10. An oily suspension of an astaxanthin derivative of the formula I comprising:
a) 1 to 20% by weight of at least one finely divided astaxanthin derivative which comprises at least 70% by weight, based on the total amount of astaxanthin derivative in the suspension, of an astaxanthin derivative of the formula I according to claim 1 or 2,
b) 80 to 99% by weight of an edible oil or a mixture of edible oils, and
c) optionally one or more oxidation stabilizers,
where the particles of the astaxanthin derivative in the suspension have a volume-average particle diameter, determined by Fraunhofer diffraction, in the range from 0.5 to 50 µm, and the data in % by weight relate to the total weight of the oily suspension.

11. An oily preparation of an astaxanthin derivative comprising:
a) 0.01 to 2.0% by weight, based on the total weight of the oily preparation, of at least one astaxanthin derivative comprising at least 70% by weight of the astaxanthin derivative of formula I according to claim 1 or 2,
b) an edible oil or a mixture of edible oils, and
c) optionally one or more oxidation stabilizers,
where the astaxanthin derivative in the edible oil is at least 90% in the dissolved form and > 80% has an all-trans configuration.

12. The use of an oily preparation according to claim 11 as additive to animal feeds, foods, food supplements, pharmaceutical compositions or cosmetic compositions.

13. A composition in the form of an animal feed, food, food supplement, pharmaceutical composition or cosmetic composition, comprising an oily preparation according to claim 11.

14. The composition according to claim 13 in the form of pellets for animal feed.

15. The composition according to claim 13 or 14 comprising the astaxanthin derivative in an amount of 10 to 100 ppm, based on the total weight of the composition.

## Revendications

1. Procédé pour la préparation d'une solution d'un dérivé d'astaxanthine dans une huile comestible, comprenant
i) la préparation d'une suspension du dérivé d'astaxanthine dans l'huile comestible, les particules du dérivé d'astaxanthine dans la suspension présentant un diamètre moyen volumique des particules, déterminé par diffraction de Fraunhofer, dans la plage de 0,5 à 50 µm, et
ii) l'incorporation de la suspension du dérivé d'astaxanthine dans une huile comestible, avec obtention d'une solution du dérivé d'astaxanthine, le dérivé d'astaxanthine étant constitué, à raison d'au moins 70% en poids, par au moins un composé de formule I 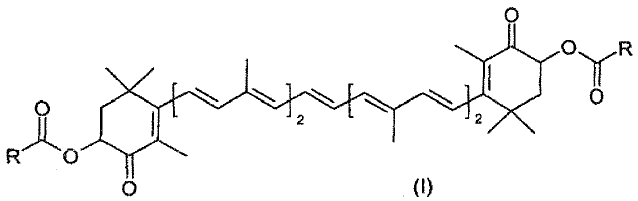 où R représente un radical de formule ; r 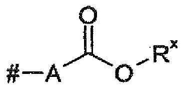 où # signifie la liaison au groupe carbonyle, A représente C₁-C₆-alkylène linéaire ou ramifié ou -CH=CH- et R^{x} représente C₁-C₁₀-alkyle.

2. Procédé selon la revendication 1, où le radical R dans la formule I représente un radical de formule 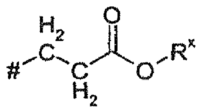 où R^{x} signifie méthyle ou éthyle.

3. Procédé selon l'une quelconque des revendications précédentes, où la suspension du dérivé d'astaxanthine dans l'huile comestible est préparée par broyage du dérivé d'astaxanthine dans l'huile comestible.

4. Procédé selon l'une quelconque des revendications précédentes, où, dans l'étape i), une température de 100°C n'est pas dépassée.

5. Procédé selon l'une quelconque des revendications précédentes, où, dans l'étape i), on utilise 1 à 20% en poids du dérivé d'astaxanthine, par rapport à la quantité totale d'huile comestible et de dérivé d'astaxanthine.

6. Procédé selon l'une quelconque des revendications précédentes, où l'huile comestible utilisée dans les étapes i) et ii) est choisie parmi l'huile de germes de soja, l'huile de palme, l'huile de palmiste, l'huile de tournesol, les huiles PUFA (acides gras polyinsaturés), les huiles MTC (triglycérides à chaîne moyenne), les huiles de poisson, l'huile de carthame, l'huile de maïs, l'huile d'olive, l'huile de graines de lin, l'huile de colza, l'huile de riz et les mélanges de ces huiles.

7. Procédé selon l'une quelconque des revendications précédentes, où l'huile comestible dans l'étape ii) présente une température dans la plage de 30 à < 100°C.

8. Procédé selon l'une quelconque des revendications précédentes, où on ajoute dans l'étape ii) la suspension sous agitation à température augmentée à l'huile comestible.

9. Procédé selon l'une quelconque des revendications 1 à 7, où on mélange dans l'étape ii) la suspension dans un mélangeur en ligne avec l'huile comestible à température augmentée.

10. Suspension huileuse d'un dérivé d'astaxanthine de formule I, constituée par :
a) 1 à 20% en poids d'au moins un dérivé finement divisé d'astaxanthine, qui est constitué à raison d'au moins 70% en poids, par rapport à la quantité totale de dérivé d'astaxanthine dans la suspension, d'un dérivé d'astaxanthine de formule I selon la revendication 1 ou 2,
b) 80 à 99% en poids d'une huile comestible ou d'un mélange d'huiles comestibles, et
c) le cas échéant un ou plusieurs stabilisateurs contre l'oxydation,
où les particules du dérivé d'astaxanthine dans la suspension présentent un diamètre moyen volumique des particules, déterminé par diffraction de Fraunhofer, dans la plage de 0,5 à 50 µm, et les indications en % en poids se rapportent au poids total de la suspension huileuse.

11. Préparation huileuse d'un dérivé d'astaxanthine, constituée par :
a) 0,01 à 2,0% en poids, par rapport au poids total de la préparation huileuse, d'au moins un dérivé d'astaxanthine, constitué à raison d'au moins 70% en poids par le dérivé d'astaxanthine de formule I selon la revendication 1 ou 2,
b) une huile comestible ou d'un mélange d'huiles comestibles, et
c) le cas échéant un ou plusieurs stabilisateurs contre l'oxydation,
où le dérivé d'astaxanthine se trouve dans l'huile comestible à raison d'au moins 90% sous forme dissoute et présente, à raison de > 80% une configuration all-trans.

12. Utilisation d'une préparation huileuse selon la revendication 11 comme additif de fourrages pour animaux, d'aliments, de compléments alimentaires, d'agents pharmaceutiques ou d'agents cosmétiques.

13. Agent sous forme d'un fourrage pour animaux, d'un aliment, d'un complément alimentaire, d'un agent pharmaceutique ou d'un agent cosmétique, comprenant une préparation huileuse selon la revendication 11.

14. Agent selon la revendication 13 sous forme de pellets pour des fourrages pour animaux.

15. Agent selon la revendication 13 ou 14, contenant le dérivé d'astaxanthine en une quantité de 10 à 100 ppm, par rapport au poids total de l'agent.
